# EUROPÄISCHE PATENTSCHRIFT

(11) **EP 0 820 808 B1**
(45) Veröffentlichungstag und Bekanntmachung des Hinweises auf die Patenterteilung: **04.09.2002**
(21) Anmeldenummer: 97111739.5
(22) Anmeldetag: 10.07.1997
(51) Int. Cl.: B01J 23/46, B01J 23/89, B01J 23/889, C07C 209/36

(54) **Multimetallischer Katalysator und Verfahren zur Herstellung substituierter aromatischer Amine**
Multi-metallic catalyst and method for producing substituted aromatic amines
Catalyseur polymétallique et procédé de fabrication d'amines aromatiques substitués

(30) Priorität: 23.07.1996 DE 19629659; 06.09.1996 DE 19636214
(43) Veröffentlichungstag der Anmeldung: 28.01.1998
(73) Patentinhaber: Degussa AG, 40474 Düsseldorf (DE)
(72) Erfinder: Auer, Emannuel, Dr., 60528 Frankfurt/Main (DE); Freund, Andreas, Dr., 63801 Kleinostheim (DE); Gross, Michael, 60385 Frankfurt (DE); Hartung, Rolf, 63543 Neuberg (DE)

(56) Entgegenhaltungen:
- EP-A- 0 356 046
- DE-A- 3 006 748
- GB-A- 1 453 966
- US-A- 4 018 670
- US-A- 4 124 538
- US-A- 4 132 672
- US-A- 4 994 247
- US-A- 5 102 851
- US-A- 5 192 452

## Beschreibung

Die vorliegende Erfindung betrifft einen Katalysator, welcher Iridium und wenigstens ein weiteres Dotierungselement auf einem Träger enthält für die Herstellung substituierter aromatischer Amine durch Hydrierung der entsprechenden substituierten Nitroaromaten.

Die Hydrierung von aromatischen Nitroverbindungen mit Halogensubstituenten - insbesondere Chlor - zur Herstellung der entsprechenden Amine ist seit langem bekannt (Ullmann, Enzyklopädie der technischen Chemie, 5. Auflage, Band A2, Seite 46 (1985)) und wird in Gegenwart von Metallkatalysatoren mit Wasserstoff als Reduktionsmittel durchgeführt. Ein zentrales Problem der Reaktion ist die unerwünschte Dechlorierung des Aromaten, was zu einer Verringerung der Ausbeute an aromatischer Aminverbindung führt. Aus diesem Grund beschäftigen sich zahlreiche Patentschriften mit Verfahren, die eine Halogenabspaltung so gering wie möglich zu halten versuchen.

So kann durch Verwendung von Platin und Ruthenium auf Trägermaterialien (US 4 760 187 A) ebenso eine Verbesserung der Selektivität erreicht werden wie durch den Einsatz von platin-, palladium-, rhodium-, iridium-, ruthenium- und osmiumhaltiger Katalysatoren, die mit sauren Phosphorverbindungen nachbehandelt werden (DE 30 06 748 A).

Aktivität und Selektivität der Edelmetallkatalysatoren können durch Anwesenheit eines Cokatalysators in der Reaktionsmischung beeinflußt werden. Gemäß der US 5,105,012 wird neben Palladium auf einem Kohlenstoffträger als Hauptkatalysator Eisenpulver oder ein Eisensalz als Cokatalysator in die Reaktionsmischung gegeben. Die US 3 253 039 A schlägt vor, zusätzlich zu einem Katalysator aus Platin auf Kohlenstoff Silbernitrat in die Reaktionsmischung zu geben. Silber ist dabei in der gesamten Reaktionsmischung verteilt, ohne mit dem Platin des Katalysators legiert zu sein. Als weitere Schwermetallzusätze wurden Blei-, Kupfer-, Nickel-, Wismut-, und Chromnitrat untersucht.

Durch gezielte Dotierung eines Edelmetallkatalysators mit verschiedenen Haupt- und Nebengruppenelementen können ebenfalls Aktivität und Selektivität der Hydrierung beeinflußt werden. So wird zum Beispiel in der DE 42 36 203 A1 vorgeschlagen, einen mit Nickel und/oder Cobalt dotierten Platinkatalysator auf Aktivkohle zu verwenden. Gemäß der DE 42 18 866 C1 wird die Selektivität der Hydrierung durch Dotieren eines Platinkatalysators auf Aktivkohle mit Kupfer verbessert. Platin und Kupfer werden dabei gleichzeitig auf dem Aktivkohleträger abgeschieden und anschließend reduziert.

Die bekannten Hydrierkatalysatoren werden zum Beispiel durch Einbringen des Trägermaterials in eine Edelmetallsalzlösung und Verdampfen des Lösungsmittels hergestellt. Gegebenenfalls wird der Katalysator anschließend reduziert. Alternativ hierzu kann das Trägermaterial auch mit den katalytisch aktiven Elementen imprägniert werden, indem das Trägermaterial mit einer Lösung dieser Elemente in Kontakt gebracht wird und die Hydroxide dieser Elemente im alkalischen Milieu ausgefällt werden. Hieran kann sich ebenfalls eine Reduktion anschließen. Weiterhin ist es bekannt, auf das Trägermaterial eine Lösung der Edelmetallsalze aufzusprühen.

Die bislang bekannten Verfahren zur Hydrierung substituierter aromatischer Nitroverbindungen weisen jedoch hinsichtlich Aktivität und Selektivität noch einige Probleme auf. So führen Metalldotierungen in Verbindung mit stickstoffhaltigen Zusätzen bei der Hydrierung zu halogenierten Azo- und Azoxybenzolderivaten (EP 0 073 105 A). Aufgrund der Toxizität solcher Verbindungen ist deren Bildung als Nebenprodukte bei der Hydrierung von Halonitroaromaten zu vermeiden.

Aufgabe der vorliegenden Erfindung ist es, einen Katalysator für die Hydrierung von substituierten Nitroaromaten anzugeben, der sich gegenüber den bekannten Katalysatoren insbesondere durch eine verbesserte Selektivität auszeichnet.

Gegenstand der Erfindung ist ein multimetallischer Katalysator enthaltend Iridium und wenigstens ein weiteres Dotierungselement auf einem Träger, dadurch gekennzeichnet, daß Iridium mit wenigstens einem Element aus der Gruppe Mangan, Cobalt, Eisen, Nickel, Kupfer und Ruthenium dotiert ist, wobei er 0,3 bis 12 Gew.-% Iridium bezogen auf das Gewicht des Trägers und bezogen auf Iridium 1 bis 100 Gew.-% der Dotierungselemente enthält und der Träger aus Aktivkohle besteht, wobei mindestens 80 Gew.-% der Aktivkohlepartikel eine Korngröße von weniger als 100 µm aufweisen.

Geeignete Trägermaterialien für den Katalysator sind Aktivkohle oder oxidische Materialien wie Aluminiumoxid, Titandioxid, Siliziumdioxid oder Mischoxide davon. Bevorzugt wird Aktivkohle verwendet. Sie kann pflanzlichen oder tierischen Ursprungs sein, und durch verschiedene Verfahren (z.B. Wasserdampf, Phosphorsäure etc.) aktiviert worden sein. Sie kann aus poröser und nicht poröser Aktivkohle bestehen, wobei vorzugsweise mindestens 80 Gew.-% der Aktivkohlepartikel eine Korngröße von weniger als 100 µm aufweisen. Derartige Aktivkohlen sind allgemein über verschiedene Anbieter zugänglich.

Der erfindungsgemäße Katalysator enthält Iridium in einer Menge zwischen 0,3 und 12, bevorzugt zwischen 0,5 und 7 Gew.-% bezogen auf das eingesetzte Trägermaterial, sowie wenigstens ein Metall aus der Gruppe Mangan, Eisen, Cobalt Nickel, Kupfer und Ruthenium, in einer Menge zwischen 1 und 100, bevorzugt zwischen 5 und 50 Gew.-%, bezogen auf Iridium.

Zur Herstellung des Katalysators wird zunächst der Träger in Wasser suspendiert und durch Zugabe von Salzlösungen der entsprechenden Metalle imprägniert. Anschließend werden die Metalle mit Hilfe eines wasserlöslichen Reduktionsmittels bei Temperaturen zwischen 0 und 100 °C reduziert. Die Reihenfolge des Zusammenfügens von Trägermaterial, Wasser, Metallsalzlösungen und Reduktionsmittel kann auch anders gewählt werden.

Mit dem erfindungsgemäßen Katalysator können substituierte Nitroaromate zu den entsprechenden aromatischen Aminen mit hoher Selektivität hydriert werden. Hierzu werden die Nitroaromaten in geeigneten Lösungsmitteln gelöst und bei erhöhter Temperatur unter Zugabe von Wasserstoff an dem Katalysator zu den Aminverbindungen hydriert.

Bevorzugt werden chlorsubstituierte aromatische Nitroverbindungen der Formel (I) hydriert.

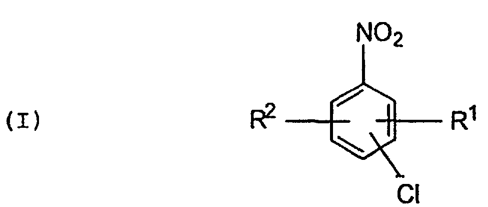

In (I) können R¹ und R² gleich oder verschieden sein und Wasserstoff, ein Alkyl-, Alkoxy-, Hydroxy-, Carboxy-, Carbonyl-, Phenyl- oder Aminoreste sowie Chlor, Fluor, Alkylcarbonylamido, Alkyloxycarbonylamido bedeuten.

Beispiele für chlorierte aromatische Nitroverbindungen gemäß Formel (I) sind 2-Nitrochlorbenzol, 3-Nitrochlorbenzol, 4-Nitrochlorbenzol, 2,4-Dichlornitrobenzol, 3,4-Dichlornitrobenzol, 2,5-Dichlornitrobenzol, 2-Chlor-4-nitrobenzol, 4-Chlor-3-nitrobenzol, 2-Chlor-4-nitroanilin, 2,3,5-Trichlornitrobenzol, 2,4,6-Trichlornitrobenzol, sowie Isomerengemische der genannten Verbindungen.

Geeignete Lösungsmittel für diese Verbindungen sind aliphatische Alkohole wie z. B. Methanol, Ethanol, Isopropanol, Butanol oder Hexanol, Carbonsäureester wie Essigsäure sowie aromatische Kohlenwasserstoffe wie Benzol, Toluol und Xylol.

Die Hydrierung kann bei Temperaturen zwischen 40 und 200, vorzugsweise zwischen 60 und 120 °C, und bei Drücken von 1 bis 200, vorzugsweise von 4 bis 150 bar, durchgeführt werden. Es ist vorteilhaft, die Hydrierung in Gegenwart einer organischen oder anorganischen basischen Verbindung durchzuführen, um gegebenenfalls abgespaltene Halogene zu binden. Vorzugsweise verwendet man hierfür Triethylamine in einer Menge von 0,05 bis 5 Gew.-% bezogen auf die eingesetzte aromatische, substituierte Nitroverbindung.

Die Hydrierung kann sowohl diskontinuierlich als auch kontinuierlich betrieben werden. Bei kontinuierlicher Fahrweise muß die Menge der umgesetzten aromatischen Nitroverbindung (Edukt) ersetzt und gegebenenfalls der verbrauchte Anteil an basischer Verbindung nachdosiert werden.

Die Hydrierung substituierter aromatischer Nitroverbindungen mit dem erfindungsgemäßen Katalysator zeichnet sich vor allem durch eine hohe Selektivität hinsichtlich möglicher Dehalogenisierungs- und anderer unerwünschter Nebenprodukte (z. B. Azoxy- und Azobenzolderivate) aus. Insbesondere die Abspaltung von Chlor kann bei Verwendung des beschriebenen multimetallischen Katalysators fast völlig vermieden werden. In vielen Fällen kann deshalb auf eine zusätzliche Nachreinigung der gewünschten chlorsubstituierten aromatischen Aminverbindung von dechlorierten Nebenverbindungen verzichtet werden. Dies ist besonders vor dem Hintergrund gestiegener Qualitätsanforderungen eine deutliche Verbesserung des Standes der Technik.

### Beispiel 1

Es wurden 94,8 g Aktivkohle in deionisiertem Wasser suspendiert. Zu dieser Suspension wurde eine Lösung von 20,74 g Hexachloroiridium(IV)säure(23%ig), 0,72 g Mangan(II)chlorid-Tetrahydrat und 0,87 g sublimiertes Eisen(III)chlorid zugefügt, mit Natronlauge alkalisch (pH 10) gestellt, bei 80°C mit einer frisch bereiteten Natriumborhydridlösung (2 Gew.-%) reduziert und nach dem Einstellen von pH 4 abfiltriert.

Der fertige Katalysator enthielt bezogen auf sein Gesamtgewicht 5,0 Gew.-% Iridium und bezogen auf Iridum 4,2 Gew.-% Mangan und 6,2 Gew.-% Eisen.

### Beispiel 2

In gleicher Weise wie in Beispiel 1 wurde ein Katalysator unter Verwendung von 20,74 g Hexachloroiridium(IV)säure-(23%ig) und 1,45 g sublimiertem Eisen(III)chlorid hergestellt.

Der fertige Katalysator enthielt bezogen auf sein Gesamtgewicht 5,0 Gew.-% Iridium und bezogen auf Iridium 10,5 Gew.-% Eisen.

### Beispiel 3

Zu einer Suspension von 94,8 g Aktivkohle in deionisiertem Wasser wurden 20,74 g Hexachloroiridium(IV)säure, 0,404 g Cobalt(II)chlorid-6-Hydrat und 1,16 g sublimiertes Eisen(III)chlorid hinzugefügt. Die Suspension wurde mit Natronlauge alkalisch (pH 10) gestellt und mit einer frisch bereiteten Natriumborhydridlösung (2 Gew-%) reduziert und nach dem Einstellen von pH 4 abfiltriert.

Der fertige Katalysator enthielt bezogen auf sein Gesamtgewicht 5,0 Gew.-% Iridium und bezogen auf Iridium 2,1 Gew.-% Cobalt und 8,4 Gew.-% Eisen.

### Beispiel 4

In gleicher Weise wie in Beispiel 3 wurde ein Katalysator unter Verwendung von 20,74 g Hexachloroiridium(IV)säure, 1,48 g Nickel(II)nitrat-Hexahydrat und 0,72 g Mangan(II)chlorid-Tetrahydrat hergestellt.

Der fertige Katalysator enthielt bezogen auf sein Gesamtgewicht 5,0 Gew.-% Iridium und bezogen auf Iridium 6,3 Gew.-% Nickel und 4,2 Gew.-% Mangan.

### Beispiel 5

In gleicher Weise wie in Beispiel 3 wurde ein Katalysator unter Verwendung von 20,74 g Hexachloroiridium (IV)säure, und 3,54 g Ruthenium(III)chlorid(14,1%ig) hergestellt.

Der fertige Katalysator enthielt bezogen auf sein Gesamtgewicht 5,0 Gew.-% Iridium und bezogen auf Iridium 10,4 Gew.-% Ruthenium.

### Beispiel 6

In gleicher Weise wie in Beispiel 3 wurde ein Katalysator unter Verwendung von 20,74 g Hexachloroiridium(IV)säure, 0,438 g Eisen(III)chlorid und 0,58 g Kupfer(II)nitrat-Tetrahydrat hergestellt.

Der fertige Katalysator enthielt bezogen auf sein Gesamtgewicht 5,0 Gew.-% Iridium und bezogen auf Iridium 3,2 Gew.-% Eisen und 3,2 Gew.-% Kupfer.

### Vergleichsbeispiel 1

Es wurde ein mit Kupfer dotierter Platinkatalysator gemäß der DE 42 18 866 C1 hergestellt.

100 g Aktivkohle wurden in 800 ml deionisiertem Wasser suspendiert. Zu dieser Suspension wurde eine Lösung von 2,5 g Hexachloroplatin(IV)säure und 0,32 g Kupferacetatmonohydrat in 200 ml Wasser zugefügt. Die Suspension wurde auf 80°C erwärmt, mit Natronlaugelösung alkalisch gestellt und mit 0,3 ml Formaldehydlösung (37 Gew-%) reduziert. Die Suspension wurde 20 min nachgerührt und dann der Katalysator abfiltriert.

Der fertige Katalysator enthielt bezogen auf sein Gesamtgewicht 1 Gew.-% Platin und bezogen auf Platin 10 Gew.-% Kupfer.

### Vergleichsbeispiel 2

Es wurde ein reiner Platinkatalysator gemäß der DE 42 18 866 C1 hergestellt. Mit Ausnahme der fehlenden Zugabe von Kupfer wurde die Herstellung gemäß Vergleichsbeispiel 1 vorgenommen.

### Vergleichsbeispiel 3

Es wurde ein reiner Iridiumkatalysator gemäß der DE 42 18 866 C1 hergestellt. Mit Ausnahme der fehlenden Zugabe von Mangan und Eisen wurde die Herstellung gemäß Beispiel 1 vorgenommen.

### Anwendungsbeispiel

Die in den vorstehenden Beispielen hergestellten Katalysatoren wurden bezüglich ihrer Aktivität und Selektivität bei der Hochdruckhydrierung von 2-Chlornitrobenzol geprüft.

Die Hydrierungen wurden in einem Rührautoklaven mit einem Volumen von 0,5 l vorgenommen. Zur Bestimmung der katalytischen Aktivität und Selektivität der Katalystoren wurden folgende Reaktionsparameter eingehalten:

| | |
|---|---|
| Aufheizdruck | 5 bar |
| Reaktionsdruck | 10 bar |
| Temperatur | 90 °C |
| Rührgeschwindigkeit | 700 rpm |

Es wurden jeweils 78 g 2-Chlornitrobenzol in Gegenwart von 0,8 mol% Triethylamin quantitativ umgesetzt. Das Ende der Reaktion ist durch einen rapiden Abfall der Wasserstoffaufnahme zu Null bestimmbar. Die Katalysator-Einwaage betrug jeweils 0,385 g. Als mögliches Dehalogenierungsprodukt kann bei dieser Reaktion Anilin gebildet werden. Der Anteil von Anilin im Produkt wurde deshalb gaschromatografisch bestimmt.

Die Ergebnisse der Untersuchungen sind in der folgenden Tabelle zusammengefaßt. Sie zeigen, daß die erfindungsgemäßen Katalysatoren sich durch eine hohe Selektivität auszeichnen.

**Tabelle:**

| Prüfung von Aktivität und Selektivität der Katalysatoren | | | |
|---|---|---|---|
| Katalysator nach Beispiel | Lösungsmittel | Hydrierzeit [min] | Anteil Anilin [GC %] |
| Beispiel 1 | Toluol | 45 | < 0,05 |
| Beispiel 1 | Methanol | 45 | < 0,06 |
| Beispiel 2 | Toluol | 50 | < 0,09 |
| Beispiel 2 | Methanol | 55 | < 0,08 |
| Beispiel 3 | Toluol | 55 | < 0,07 |
| Beispiel 4 | Toluol | 60 | < 0,3 |
| Beispiel 5 | Toluol | 40 | < 0,2 |
| Beispiel 6 | Toluol | 55 | < 0,15 |
| V-Beispiel 1 | Toluol | 30 | 0,9 |
| V-Beispiel 2 | Toluol | 25 | 1,2 |
| V-Beispiel 3 | Toluol | 45 | 0,2 |

## Patentansprüche

1. Multimetallischer Katalysator enthaltend Iridium und wenigstens ein weiteres Dotierungselement auf einem Träger,
**dadurch gekennzeichnet,**
**daß** Iridium mit wenigstens einem Element aus der Gruppe Mangan, Cobalt, Eisen, Nickel, Kupfer und Ruthenium dotiert ist, wobei er 0,3 bis 12 Gew.-% Iridium bezogen auf das Gewicht des Trägers und bezogen auf Iridium 1 bis 100 Gew.-% der Dotierungselemente enthält und der Träger aus Aktivkohle besteht, wobei mindestens 80 Gew.-% der Aktivkohlepartikel eine Korngröße von weniger als 100 µm aufweisen.

2. Verfahren zur Herstellung substituierter aromatischer Amine durch Hydrieren der entsprechenden substituierten Nitroaromaten,
**dadurch gekennzeichnet,**
**daß** die Hydrierung an einem Katalysator gemäß dem Ansprüch 1 vorgenommen wird.

## Claims

1. A multimetallic catalyst containing iridium and at least one other doping element on a support,
**characterised in that**
the iridium is doped with at least one element from the group manganese, cobalt, iron, nickel, copper and ruthenium, wherein it contains 0.3 to 12 wt.% of iridium, with reference to the weight of the support, and 1 to 100 wt.% of doping elements, with reference to iridium, and the support consists of activated carbon, wherein at least 80 wt.% of the activated carbon particles have a particle size of less than 100 µm.

2. A process for preparing substituted aromatic amines by hydrogenation of the corresponding substituted nitroaromatic compounds,
**characterised in that**
hydrogenation is performed on a catalyst in accordance with claim 1.

## Revendications

1. Catalyseur multimétallique contenant de l'iridium et au moins un autre élément de dopage sur un support,
**caractérisé en ce que**
l'iridium est dopé par au moins un élément choisi dans le groupe constitué par le manganèse, le cobalt, le fer, le nickel, le cuivre et le ruthénium, le catalyseur contenant de 0,3 à 12 % en poids d'iridium par rapport au poids du support et de 1 à 100 % en poids de l'élément de dopage par rapport à l'iridium, et le support consistant en charbon actif, au moins 80 % en poids des particules de charbon actif ayant une taille de grain de moins de 100 µm.

2. Procédé pour la préparation d'amines aromatiques substituées, par hydrogénation des composés aromatiques nitrés substitués correspondants,
**caractérisé en ce que**
l'hydrogénation est effectuée sur un catalyseur selon la revendication 1.
